# EUROPEAN PATENT APPLICATION

(11) **EP 4 234 492 A1**
(43) Date of publication of application: **30.08.2023**
(21) Application number: 22305196.2
(22) Date of filing: 23.02.2022
(51) Int. Cl.: C01B 33/40, A61K 8/00, A61Q 17/00, A61Q 19/00

(54) **MODIFIED CLAY**

(71) Applicant: ImerTech SAS, 75015 Paris (FR)
(72) Inventor: Remia, Elodie, 31300 Toulouse (FR)
(74) Representative: Haseltine Lake Kempner LLP

(57) **Abstract**

The present invention relates to a modified clay comprising a clay and a phospholipid, a method of preparing such modified clays, their use, and cosmetic formulations comprising said modified clay.

## Description

### FIELD OF THE INVENTION

The present invention relates to a modified clay comprising a clay and a phospholipid, a method of preparing such modified clays, their use, and cosmetic formulations comprising said modified clay.

### BACKGROUND OF THE INVENTION

Modified clay is used in a range of different products, such as, cosmetics, paints, detergent, refractory varnish, thixotropic fluids, and adsorbents of organic pollutants in soil, water and air. Known modified clays, also frequently referred to as organoclays, are often produced via an organophylization process. In an organophylization process, hydrophilic natural clay can be treated with, for example, a synthetic organic compound, such as a quaternary ammonium, to produce a lipophilic modified clay.

There is a drive to move away from chemical treatments, such as the use of synthetic quaternary ammonium, to produce modified clays that comprise natural materials. This move to natural materials is in part based on the increasing need to use products that are environmentally friendly. However, the replacement of synthetic components is also driven by drawbacks associated with the products comprising such modified clays. For example, modified clays are widely used in cosmetics, and compounds such as synthetic quaternary ammonium can lead to skin irritation. The replacement of synthetic quaternary ammonium with naturally sourced compounds, whilst maintaining the desirable modification of clay remains a challenge.

It is therefore desirable to provide alternative modified clay. In particular, it is desirable to provide modified clay using a naturally sourced compound.

### SUMMARY OF THE INVENTION

The present invention is defined in the appended claims.

The present invention provides a modified clay comprising a clay and a phospholipid, wherein the clay comprises a swellable clay; and the modified clay comprises less than about 20 cmol/kg of cations not including sodium cations.

The invention further provides a method of preparing a modified clay using an organophylization process, comprising the treatment of clay with a phospholipid, wherein the clay comprises swellable clay, and wherein the weight ratio of phospholipid to swellable clay is between about 0.30 to about 1.20.

In a further embodiment the invention provides a modified clay according to such method.

The invention further provides the use of such modified clay as a thickener.

In a further embodiment the invention provides a cosmetic composition comprising such modified clay.

Certain embodiments of the present invention may provide one or more of the following advantages:
- desired viscosity of an organic phase comprising the modified clay;
- desired swelling index of the modified clay in an organic phase;
- desired ease of production;
- desired environmental credentials;
- desired compatibility in cosmetic products;
- desired suspending power of powders in an organic phase comprising the modified clay;
- desired cost of production.

The details, examples and preferences provided in relation to any particular one or more of the stated aspects of the present invention apply equally to all aspects of the present invention. Any combination of the embodiments, examples and preferences described herein in all possible variations thereof is encompassed by the present invention unless otherwise indicated herein, or otherwise clearly contradicted by context.

### BRIEF DESCRIPTION OF THE DRAWINGS

The disclosure will further be illustrated by reference to the following figures:
- Fig. 1: Schematic of a dry organophilization process
- Fig. 2: Schematic of a wet organophilization process

It is understood that the following description and references to the figures concern exemplary embodiments of the present invention and shall not be limiting the scope of the claims.

### DETAILED DESCRIPTION

The present invention is based on the surprising finding that phospholipids can be used to treat clay to obtain a modified clay product with desired properties. The use of phospholipids to treat clay, wherein the clay comprising a swellable clay. The use of such clays allows modified clays to be obtained without the use of synthetic compounds such as quaternary ammonium.

In some embodiments, the clay portion of the modified clay comprises swellable clay in an amount of about 70 wt.% or more, about 71 wt.% or more, about 72 wt.% or more, about 73 wt.% or more, about 75 wt.% or more, about 78 wt.% or more, about 80 wt.% or more, about 82 wt.% or more, about 85 wt.% or more, about 88 wt.% or more, about 90 wt.% or more, about 92 wt.% or more, about 95 wt.% or more, about 98 wt.% or more, about 99 wt.% or more, based on the total weight of clay.

The amount of swellable clay present in the modified clay as a proportion of the untreated clay can be determined using any known method. For example, in one method, a calcination step is carried out on the modified clay to eliminate the organic compound and to isolate the clay. For determination of the clay composition, powder X-ray diffraction with Rietveld refinement was carried out (Profex BGMN 4.3.5). Refinement of the spectra started at 8° 2Θ to avoid interpretation of clay mineral basal reflections. Spectra was obtained using Cu Ka radiation. No internal standards were used for quantification of XRD patterns.

In some embodiments, the modified clay comprises clay and a phospholipid, where the clay comprises a swellable clay. In some embodiments the weight ratio of the phospholipid over the swellable clay is between about 0.30 to about 1.20. In some examples, the weight ratio of the phospholipid over the swellable clay is about 0.35 to about 1.15, about 0.40 to about 1.10, about 0.45 to about 1.05, about 0.50 to about 1.00, about 0.55 to about 0.95, about 0.60 to about 0.90, about 0.65 to about 0.85, or about 0.70 to about 0.80.

The weight of phospholipid can be measured by any known method. It may be measured by measuring the phosphore thanks to a colorimetric dosage method, for example as disclosed in the PhD thesis of T. Bardeau, Phospholipides biosourcés riches en acides gras omega 3 pour la formulation de liposomes, Dec 2015.

According to the present disclosure, the modified clay comprises less than about 20 cmol/kg of cations not including sodium cations. In other words, the addition of the amounts of all cations in the modified clay, except sodium cations, results in less than 20 cmol/kg of cations in the modified clay (not including sodium cations). For example, the modified clay comprises less than about 18 cmol/kg, less than about 16 cmol/kg, less than about 14 cmol/kg, less than about 12 cmol/kg, less than about 10 cmol/kg, less than about 8 cmol/kg, less than about 6 cmol/kg, less than about 4 cmol/kg, less than about 2 cmol/kg of cations (not including sodium cations). In some embodiments the modified clay is free from cations (not including sodium cations).

The cations (not including sodium cations) are determined using any method known in the art. For example, the amount of cations can be determined by first establishing the Cation Exchange Capacity (CEC) of the samples, which is tested using a Cu-Triethylenetetramine solution buffered with CaCO₃. In a CaCO₃ buffered solution, dissolution of carbonates should be prevented. For CEC determination:
0.2 g of material was dispersed in 30 ml of 3,33 mmol/l Cu-Triethylenetetramine solution;
Samples are tested in double determination.

UV-ViS spectrophotometry was applied for analyzing Cu-Triethylenetetramine concentration, as described in Decher et al., A new Measure for active clay in green sand, as published by the American Foundry Society in 2020.

After cation exchange, soluble and exchangeable cations were determined from the exchange solution as follows:
∘ Na⁺- and K⁺-ions by flame photometry (F-AES).
∘ Ca²⁺- and Mg²⁺-ions by ICP-OES.

In some examples, the cations in the modified clay (without sodium cations) may be one or more of magnesium, calcium, and potassium.

In a preferred embodiment, the modified clay has a Cation Exchange Capacity (CEC) of no more than 50 cmol/kg. For example, the modified clay has a Cation Exchange Capacity (CEC) of no more than 45 cmol/kg, or for example, no more than 40 cmol/kg. Preferably, the modified clay has a Cation Exchange Capacity (CEC) of no more than 35 cmol/kg, or even no more than 30 cmol/kg. More preferably, the modified clay has a Cation Exchange Capacity (CEC) of no more than 27 cmol/kg, or no more than 26 cmol/kg.

Preferably, the modified clay has a Cation Exchange Capacity (CEC) of at least 1 cmol/kg, for example at least 5 cmol/kg.

For example, the modified clay has a Cation Exchange Capacity (CEC) of at least 1 cmol/kg and no more than 50 cmol/kg. Preferably the modified clay has a Cation Exchange Capacity (CEC) of at least 1 cmol/kg and no more than 45 cmol/kg, or of at least 5 cmol/kg and no more than 27 cmol/kg.

In some examples, the modified clay comprises less than 10 wt.% Fe₂O₃, less than 8 wt.% of Fe₂O₃, less than 6 wt.% Fe₂O₃, less than 4 wt.% Fe₂O₃, or less than 2 wt.% Fe₂O₃, based on the total weight of the modified clay. The amount of Fe₂O₃ in the modified clay has been determined by X-ray fluorescence analysis (XRF) after preparing a fused Li-tetraborate sample, as described ISO-12677. In summary, the powdered sample is fused with a flux to destroy its mineralogical and particulate composition. The resultant melt is cast into the shape of a glass bead which is then introduced into an XRF spectrometer. The intensities of the fluorescent X-rays of the required elements in the bead are measured and the chemical composition of the sample is analysed by reference to previously determined calibration graphs and applying corrections for inter-element effects. The calibration equations and inter-element corrections are established from beads produced from Certified Reference Materials (CRMs).

The LOI (Loss on Ignition) might be needed to perform the X-ray fluorescence analysis. The LOI refers to the mass loss of a combustion residue whenever it is heated in an air or oxygen atmosphere to high temperatures. The LOI is expressed as a weight percentage of the dry mass (dried at 110±10°C). The dried test sample is then heated in a furnace to a constant mass at (1000 ± 50°C). The difference in mass before and after the ignition process is used to calculate the loss on ignition.

### Clay

In some embodiments, the swellable clay is selected from smectite clay, a trioctahedral smectite clay or a dioctahedral smectite clay, wherein the trioctahedral smectite clay is selected from one or more of saponite, hectorite or laponite, and the dioctahedral smectite clay is selected from one or more of montmorillonite, bentonite, nontronite, or beidelleite. A swellable clay is generally understood to be a clay that is prone to large volume changes in water, in particular to volume increases.

In some embodiments, the clay comprises non-swellable components. Non-swellable components are generally understood not to increase its volume in water. In some embodiments the non-swellable components are selected from feldspar, quartz, opal, mica, kaolinite, calcite and mixtures thereof.

In some embodiments, the clay portion of the modified clay comprises non-swellable clay in an amount of about 27 wt.% or less, about 25 wt.% or less, about 22 wt.% or less, about 20 wt.% or less, about 18 wt.% or less, about 15 wt.% or less, about 12 wt.% or less, about 10 wt.% or less, about 8 wt.% or less, about 5 wt.% or less, about 3 wt.% or less, about 2 wt.% or less, about 1 wt.% or less, based on the total weight of clay.

### Phospholipid

In some embodiments the phospholipid is between two sheets of clay to form the modified clay. In some embodiments the distance between the two sheets of clay is at least 20 angstroms. For example, the distance (basal spacing d001) between the two sheets of clay is at least 25 angstroms, at least 26 angstroms, at least 27 angstroms, at least 28 angstroms, at least 29 angstroms, at least 30 angstroms, at least 35 angstroms. In some examples, the distance between the two sheets of clay is no more than 40 angstroms. The distance between the two sheets can be measured by XRD.

Phospholipids according to the present disclosure may be selected from a cationic phospholipid, a zwitterionic phospholipid, a non-ionic phospholipid or combinations thereof. In some embodiments, the phospholipid is a zwitterionic phospholipid.

Phospholipids are a class of lipids that comprise a hydrophilic "head" portion and one or more hydrophobic "tail" portions. The phospholipid may comprise one hydrophilic tail portion. Alternatively, the phospholipid may comprise two hydrophilic tail portions.

The hydrophobic tail portions may be substituted or unsubstituted alkyl chains with at least one carbon atom. In some embodiments the hydrophobic tail is derived from fatty acids.

In some embodiments, the hydrophilic head portion may comprise a quaternary ammonium group. In some embodiments, the nitrogen atom of the quaternary ammonium group is attached to at least two methyl groups. In some embodiments, the nitrogen atom of the quaternary ammonium group is attached to three methyl groups. In some embodiments, the nitrogen atom of the quaternary ammonium group is attached to at least two hydrogen atoms. In some embodiments, the nitrogen atom of the quaternary ammonium group is attached to three hydrogen atoms.

Without wishing to be bound by theory, it is considered that in the case of a cationic phospholipid and a zwitterionic phospholipid, the hydrophilic head portion links to the clay resulting in the modified clay.

In some embodiments the phospholipid is phosphatidylcholine, phosphatidylethanolamine, or phosphatidylserine. In some embodiments, the phospholipid is phosphatidylcholine. In some embodiments, the phospholipid is phosphatidylethanolamine. In some embodiments, the phospholipid is phosphatidylserine.

In some embodiments, the phospholipid is from a natural source. In some embodiments the phospholipid is from lecithin and the lecithin is from a natural source. In some embodiments the phospholipid may be from one or more vegetable source, one or more animal source, or a mixture thereof.

In some embodiments the vegetable source is selected from the oils of soybean, sunflower, rapeseed, corn, rice bran, cottonseed, barley, flaxseed, jangli badam seed, palash seed, papaya seed, peanut, sesame, cacao beans, carrot seeds, coriander see, oats, durum wheat, walnut, niger seed, avocado fruit, olive fruit, garlic palm, cucurbirt, camelina, hemp and mixtures thereof.

In some embodiments, the animal source is selected from egg yolk, such as: duck egg yolk, goose egg yolk, quail egg yolk, turkey egg yolk, ostrich egg yolk; milk, such as cow's milk, ewe's milk, goat milk; bovine brain.

In some embodiments the phospholipid is lecithin, and the natural source is selected from soyabean, sunflower oil, rapeseed oil, or mixtures thereof.

### Method

The method of preparing a modified clay according to the present invention involves utilising the organophylization process. The organophylization used herein is a process typically used to chemically modify natural clays with surfactants to obtain hydrophobic clays. In the present invention, the organophylization process is used to provide a modified clay by treating clay with a phospholipid. In some embodiments, the weight ratio of phospholipid to swellable clay is between about 0.30 to about 1.20. In some examples, the weight ratio of phospholipid to swellable clay is about 0.35 to about 1.15, about 0.40 to about 1.10, about 0.45 to about 1.05, about 0.50 to about 1.00, about 0.55 to about 0.95, about 0.60 to about 0.90, about 0.65 to about 0.85, or about 0.70 to about 0.80.

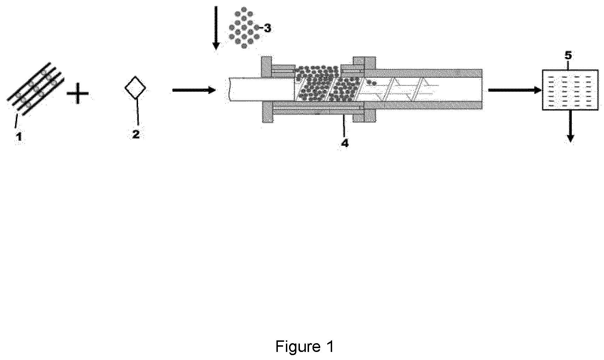

In one embodiment the organophylization process is a dry process. For example, the dry process can be carried out as depicted in Fig. 1. The clay (1) is mixed with the phospholipid (2) to which small amounts of water (3) are added (to reach about 30 wt.% water in the mixture). This mixture with water is then processed in an extruder (4), and then left about 6 hours in a closed bag (to prevent any water evaporation) at 30°C. The resulting modified clay is dried in an oven (5) at 60°C until the water content is less than about 5 wt.% based on the total weight of the modified clay. Then the modified clay is milled in a grinder until having less than 30%wt. particles > 75µm (200 mesh) as measured by sieving. Any known sieving method can be used, for example, Alpine Augsburg A200L, 1978, Nr.158306.

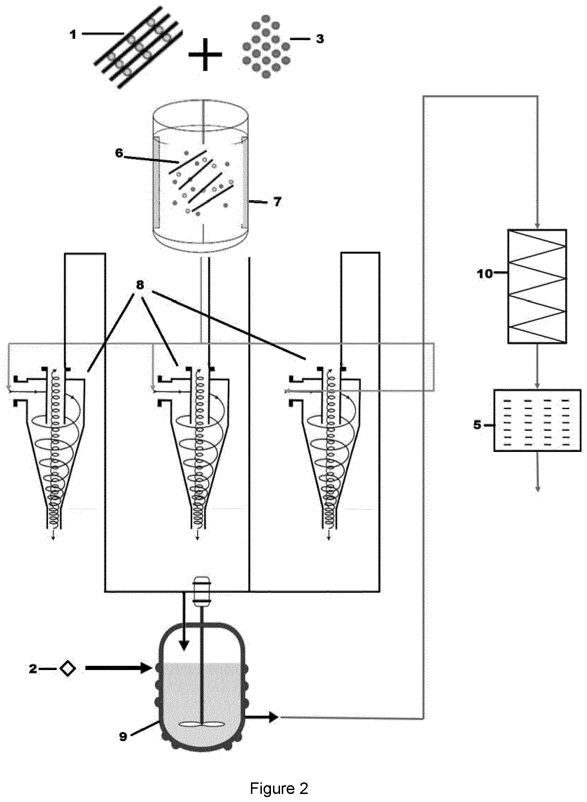

In one embodiment the organophylization process is a wet process. For example, the wet process can be carried out as depicted in Fig. 2. The clay (1) is dispersed in water (3) to form a suspension (6) (e.g. 2-5 wt.% clay suspension in water) and agitated using a mechanical agitator (7). The resulting suspension is fed through one or more hydrocyclones (8), wherein the coarse solids are expelled at the bottom of the hydrocyclones and the liquids and fine solids are expelled at the top of the hydrocyclones. The liquid and fine solids are collected in a stirring chamber (9) and mixed with the phospholipid (2) (e.g. 5-20 wt.% at 60°C). The floated, hydrophobic flocculated mass is then filtered through a filtration device (10) before being dried in an oven (5) until the moisture level is less than about 5 wt.% based on the total weight of the modified clay. Then the modified clay is milled in a grinder until having less than 30%wt. particles > 75µm (200 mesh) as measured by sieving. Any known sieving method can be used, for example, Alpine Augsburg A200L, 1978, Nr.158306.

In one embodiment, the organophylization process is a dry process and carried out by mixing the clay and the phospholipid; and treating the mixture at a temperature of about 30 °C to about 70°C for at least 1 h.

In some embodiments the steps of the organophylization process are carried out sequentially and in some embodiments the steps of the organophylization process are carried out simultaneously. For example, in a sequentially process, the mixing is carried out in the first step and the treating is carried out in a second step. For example, in a simultaneous process, both the mixing step and the treating step are carried out at the same time at a specific temperature.

In some embodiments, the heat treatment step does not change the moisture content of the mixture. In some embodiments the heat treatment is performed at a constant moisture content. For example, the heat treatment may be carried out in a closed system to ensure no loss of moisture. Such a closed system may be, for example, a closed vial or a closed plastic bag.

In some embodiments, the mixture is heat treated at about 30 °C, about 35 °C, about 40 °C, about 45 °C, about 50 °C, about 55°C, about 60°C, or about 65°C. In some examples, the mixture is heat treated for about 1 h to about 24 h, about 2 h to about 24 h, about 3 h to about 24 h, about 4 h to about 23 h, about 5 h to about 22 h, about 6 h to about 21 h, about 7 h to about 20 h, about 8 h to about 19 h, about 9 h to about 18 h, about 10 h to about 17 h, about 11 h to about 16 h, about 12 h to about 15 h, about 11 h to about 14 h, or about 12 h to about 13 h. For example, the mixture is treated at 30 °C for about 3 h, about 4 h, about 5 h, about 6 h, about 7 h, about 8 h, about 9 h, about 10 h, about 11 h, about 12 h, about 13 h, about 14 h, about 15 h, about 16 h, about 17 h, about 18 h, about 19 h, about 20 h, about 21 h, about 22 h, about 23 h, about 24 h. For example, the mixture is treated at 40 °C for about 3 h, about 4 h, about 5 h, about 6 h, about 7 h, about 8 h, about 9 h, about 10 h, about 11 h, about 12 h, about 13 h, about 14 h, about 15 h, about 16 h, about 17 h, about 18 h, about 19 h, about 20 h, about 21 h, about 22 h, about 23 h, about 24 h. For example, the mixture is treated at 50 °C for about 3 h, about 4 h, about 5 h, about 6 h, about 7 h, about 8 h, about 9 h, about 10 h, about 11 h, about 12 h, about 13 h, about 14 h, about 15 h, about 16 h, about 17 h, about 18 h, about 19 h, about 20 h, about 21 h, about 22 h, about 23 h, about 24 h.

In some embodiments, the method according to the invention comprises a drying step. In some embodiments, a drying step is carried out to obtain a product with a moisture content of about 5 wt.% or less, based on the total weight of the product. For example, a drying step is carried out to obtain a product with a moisture content of about 4.5 wt.% or less, about 4.0 wt.% or less, about 3.5 wt.% or less, about 3.0 wt.% or less, about 2.5 wt.% or less, about 2.0 wt.% or less, about 1.5 wt.% or less, or about 1.0 wt.% or less, based on the total weight of the product. In some examples, a drying step is carried out to obtain a moisture content of about 0.5 wt.%, about 0.5 wt.% or more, about 0.6 wt.%, about 0.6 wt.% or more, about 0.7 wt.%, about 0.7 wt.% or more, about 0.8 wt.%, about 0.8 wt.% or more, about 0.9 wt.%, about 0.9 wt.% or more based on the total weight of the product. In some examples, the moisture content is between about 5 wt.% and 0.5 wt.% based on the total weight of the product.

In some embodiments the drying step is carried out at a temperature of about 30 °C to about 70 °C. For example, the drying step is carried out at a temperature of about 35 °C to about 65 °C, about 40 °C to about 60 °C, or about 45 °C to about 55 °C. For example, the drying step is carried out at a temperature of about 35 °C, about 40 °C, about 45 °C, about 50 °C, about 55 °C, about 60 °C, or about 65 °C.

### Use

In some embodiments the modified clay according to the invention is used as a thickener, wherein the thickener is an organic phase thickener. In some examples the organic phase thickener is a cosmetic organic phase thickener.

In some embodiments, the cosmetic formulation is foundation, nail polish, deodorant, antiperspirant, sun care product, mascara, eyeliner and/or lipstick. The cosmetic formulation may comprise an oil. The oil used may be any oil that is suitable for use in a cosmetic formulation. Possible oils include, but are not limited to, coco-caprylate/caprate, dicaprylyl ether, C11-C13 alkane, caprylic/capric triglyceride, macadamia ternifolia seed oil, C9-C12 alkane, isododecane, isohexadecane, C15-C19 alkane, dimethicone 2cst, dimethicone 5cst.

The details, examples and preferences provided in relation to any particular aspect of the present invention apply equally to all aspects of the present invention. Any combination of the embodiments, examples and preferences described herein in all possible variations thereof are encompassed by the present invention unless otherwise indicated herein, or otherwise clearly contradicted by context.

In certain embodiments, the modified clay, method of preparing the modified clay and/or use of the modified clay may have one or more of the following effects:
- good swelling in oil;
- good thixotropic behaviour;
- decrease chemical waste;
- good biodegradability of the organic part of the modified clay;
- decrease risk for the environment, in particular aquatic environment;
- reduced irritation to skin.

The present disclosure may be described by one or more of the following numbered paragraphs:
1. A modified clay comprising a clay and a phospholipid,
   wherein the clay comprises a swellable clay; and the modified clay comprises less than about 20 cmol/kg of cations not including sodium cations.
2. A modified clay according to paragraph 1, wherein the weight ratio of the phospholipid over the swellable clay is between about 0.30 to about 1.20.
3. A modified clay comprising a clay and a phospholipid,
   wherein the clay comprises a swellable clay; wherein the weight ratio of the phospholipid over the swellable clay is between about 0.30 to about 1.20, and the modified clay comprises less than about 20 cmol/kg of cations not including sodium cations.
4. The modified clay according to any of paragraphs 1 to 3, wherein the modified clay has an X-ray diffractogram comprising at least the following characteristic diffraction peak:
   a basal spacing (001) located at a distance of the order of 25-40 Å, representing said swelling mineral phase, which peak has an intensity of at least 8000.
5. The modified clay according to any of paragraphs 1 to 4, wherein the modified clay has a Cation Exchange Capacity (CEC) of no more than 50 cmol/kg.
6. The modified clay according to any of paragraphs 1 to 5, wherein the wherein the clay comprises a swellable clay in an amount of about 70 wt.% or more based on the total weight of clay.
7. The modified clay according to any of paragraphs 1 to 6, wherein the swellable clay is selected from smectite clay, a trioctahedral smectite clay or a dioctahedral smectite clay.
8. The modified clay according to paragraph 7, wherein the trioctahedral smectite clay is selected from one or more of saponite, hectorite or laponite.
9. The modified clay according to paragraph 7, wherein the dioctahedral smectite clay is selected from one or more of montmorillonite, bentonite, nontronite, or beidelleite.
10. The modified clay according to any preceding paragraph, wherein the clay comprises non-swellable components.
11. The modified clay according to paragraph 10, wherein the non-swellable components are selected from feldspar, quartz, opal, mica, kaolinite, calcite and mixtures thereof.
12. The modified clay according to paragraph 10 or paragraph 11, wherein the non-swellable components are present in the clay in an amount of about 27 wt.% or less based on the total weight of the clay.
13. The modified clay according to any preceding paragraph, wherein the phospholipid is between two sheets of clay.
14. The modified clay according to paragraph 13, wherein the distance between two sheets of clay is at least 20 angstroms.
15. The modified clay according to any preceding paragraph, where the phospholipid may be selected from a cationic phospholipid, a zwitterionic phospholipid, a non-ionic phospholipid or combinations thereof.
16. The modified clay according to any preceding paragraph, where the phospholipid comprises at least one hydrophilic head portion and at least one hydrophobic tail portion.
17. The modified clay according to paragraph 16, wherein the phospholipid comprises at least two hydrophobic tail portions.
18. The modified clay according to paragraph 16 or paragraph 17, wherein the hydrophilic head portion comprises a quaternary ammonium group.
19. The modified clay according to paragraph 18, wherein the nitrogen atom of the quaternary ammonium group is attached to at least two methyl groups.
20. The modified clay according to paragraph 18 or paragraph 19, wherein the nitrogen atom of the quaternary ammonium group is attached to three methyl groups.
21. The modified clay according to paragraph 18, wherein the nitrogen atom of the quaternary ammonium is attached to at least two hydrogen atoms.
22. The modified clay according to paragraph 18 or paragraph 21, wherein the nitrogen atom of the quaternary ammonium group is attached to three hydrogen atoms.
23. The modified clay according to any preceding paragraph, wherein the phospholipid is phosphatidylcholine, phosphatidylethanolamine, or phosphatidylserine.
24. The modified clay according to any preceding paragraph, wherein the phospholipid is phosphatidylcholine.
25. The modified clay according to any preceding paragraph, wherein the phospholipid is from a natural source.
26. The modified clay according to any preceding paragraph, wherein the phospholipid is from lecithin and the lecithin is from a natural source.
27. The modified clay according to paragraph 25 or paragraph 26, wherein the natural source is selected from one or more vegetable source, one or more animal source or mixtures thereof.
28. The modified clay according to any preceding paragraph, wherein the modified clay comprises less than 10 wt.% of Fe₂O₃, based on the total weight of the modified clay.
29. A method of preparing a modified clay using an organophylization process, consisting in treating a clay with a phospholipid, the clay comprising swellable clay, and wherein the weight ratio of the phospholipid to swellable clay is between about 0.30 to about 1.20.
30. The method according to paragraph 29, wherein the organophylization process is a dry process.
31. The method according to paragraph 29, wherein the organophylization process is a wet process.
32. The method according to any one of paragraphs 29 to 30, wherein the organophylization process is carried out by mixing the clay and the phospholipid; and heat treating the mixture at a temperature of about 30 °C to about 70°C for at least 1 h.
33. The method according to paragraph 32, wherein the mixing step and the treating step are carried out sequentially.
34. The method according to paragraph 32, wherein the mixing step and the treating step are carried out simultaneously.
35. The method according to paragraph 32, wherein the mixing is carried out at a constant moisture content.
36. The method according to any one of paragraphs 32 to 35, wherein the mixture is treated for about 3 h to about 24 h.
37. The method according to any one of paragraphs 29 to 36, wherein the method further comprises a drying step.
38. The method according to paragraph 37, where the drying step is carried out to obtain a product with a moisture content of about 5 wt.% or less, based on the total weight of the product.
39. The method according to paragraph 37 or paragraph 38, wherein the drying step is carried out to obtain a product with a moisture content of about 0.5 wt.% or more, based on the total weight of the product.
40. The method according to any one of paragraphs 37 to 39, wherein the drying step is carried out at a temperature of about 30 °C to about 70 °C.
41. The method according to any one of paragraphs 29 to 40, wherein the phospholipid comprises phosphatidylcholine or phosphatidylethanolamine, or phosphatidylserine.
42. A modified clay obtained according to the method of any one of paragraphs 29 to 41.
43. Use of the modified clay according to any one of paragraphs 1 to 28 or paragraph 42 as a thickener.
44. The use according to paragraph 43, wherein the thickener is an organic phase thickener.
45. The use according to paragraph 44, wherein the organic phase thickener is an oil thickener.
46. The use according to any one of paragraphs 43 to 45 in a cosmetic formulation.
47. The use according to paragraph 46, wherein the cosmetic formulation is foundation, nail polish, deodorant, antiperspirant, sun care product, mascara, eyeliner and/or lipstick.
48. A cosmetic composition comprising the modified clay of any one of paragraphs 1 to 28 or paragraph 41.
49. The cosmetic composition of paragraph 48 for use in a cosmetic formulation.
50. The cosmetic composition of paragraph 49, wherein the cosmetic formulation is foundation, nail polish, deodorant, antiperspirant, sun care product, mascara, eyeliner and/or lipstick.

### EXAM PLES

In the following examples the clay used was bentonite (B) with a smectite content of 74% and 14 cmol/kg of cations not including sodium cations. B is made up of the following (Table 1, as measured by XRF):

**Table 1: Bentonite (B)**

| | | **Si0₂** | **Fe20₃** | **Ti0₂** | **Al₂0₃** | **Mg0** | **Ca0** | **Na₂0** | **K₂0** | **P₂0₅** | **LOI** |
|---|---|---|---|---|---|---|---|---|---|---|---|
| Bentonite (B) (wt.%) | | 58.8 | 2.3 | 0.23 | 23.4 | 2.4 | 1.5 | 3.5 | 0.2 | 0.03 | 5.9 |

The phospholipid used was soy lecithin (L), comprising different percentages of phosphatidylcholine (PC) as shown in Table 2.

B was treated with varying amounts of L using the dry organophylization process according to Figure 1. The modified clays (MC, organoclays) obtained are shown in Table 2:

**Table 2: Viscosity and Swelling Index of Modified Clays (MC)**

| **Modified Clay (MC)** | **% of PC** | **Amount of L per 100g of B (g)** | **T (°C)** | **Viscosity 10% in polar oil (t24h)** | **Swelling index in polar oil (ml/2g)** | **CEC (cmol/kg)** | **Na⁺ (cmol/kg)** | **Ca²⁺ (cmol/kg)** | **Mg²⁺ (cmol/kg)** | **K+ (cmol/kg)** | **ΣCations (except Na⁺)** |
|---|---|---|---|---|---|---|---|---|---|---|---|
| Comp Ex. 1 | 13 | 60 | TA | 37Po (S1) | 6 | 53 | 38 | 6 | 10 | 15,8 | 32 |
| Comp Ex. 2 | 13 | 90 | TA | 50Po (S1) | 7 | 38 | 31 | 2 | 10 | 19 | 31 |
| Comp Ex. 3 | 13 | 90 | 40°C | 60,8Po (S1) | 7 | 40 | 31 | 1 | 11 | 19 | 30 |
| Ex. 4 | 37 | 90 | 40°C | 364 Po (S2) | 9 | 42 | 46 | <0.1 | 2.5 | 7.9 | 10,5 |
| Ex. 5 | 90 | 90 | 40°C | 746,9Po (S2) | 11 | 26 | 45 | 0 | 2 | 1.3 | 3.3 |

The viscosity in Table 2 was measured using a Viscometer QC-100 (Anton Paar) and carrying out the following procedure:
1 - Make a pre-mix ethanol and demineralized water (95/5 ethanol/demineralized water)
2 - Under ultra turrax, add organoclay (total 10% in formula) in oil phase (total 85% in formula) at 50°C during 20min
3 - Add pre-mix ethanol/demineralized water (total 5% in formula) at 50°C during 10min
4 - Wait for the formula (10g organoclay in 85g of oil + 5gEtOh/water in 150ml beaker) cool down during 24h
5 - Measure the viscosity (in Po) by a viscometer at low shear rate (0.3RPM) after 24h with the suitable spindle (called S1, S2, S3 in the table - S1 is preferable for viscosities up to 100 Po, S2 is preferable for viscosities up to 1000 Po, S3 for viscosities up to 2000 Po) depending on the viscosity of the product.

The swelling index in Table 2 was measured by taking 2g of MC in 100ml of polar oil (caprilic/capric triglycerides oil) in a graduated cylinder. The volume of the MC that swelled is noted after 24h in mL/2g.

A good swelling index is considered to be 7 or more. A good viscosity is considered to be 350 Po or more. As can be seen from Table 2 only Example 4 (Ex.4) and example 5 (Ex.5) demonstrate a combination of good viscosity and a good swelling index.

**Table 3: Amounts and percentages of phosphatidylcholine and swellable clay of the Modified Clays (MC)**

| **Modified Clay (MC)** | **Amount of L or 100g of B (g)** | **Amount of L in 100g MC (g)** | **% of PC in L** | **% of PC in MC** | **Amount of clay in 100g MC (g)** | **% swellable clay in clay** | **% swttllable clay in MC** | **Ratio PC/swellable** |
|---|---|---|---|---|---|---|---|---|
| Comp Ex. 1 | 60 | 37.5 | 13 | 4.8 | 62.5 | 72 | 45 | 0.06 |
| Comp Ex. 2 | 90 | 47.3 | 13 | 6.1 | 52.7 | 72 | 38 | 0.16 |
| Comp Ex. 3 | 90 | 47.3 | 13 | 6.1 | 52.7 | 72 | 38 | 0.16 |
| Ex. 4 | 90 | 47.3 | 37 | 17.5 | 52.7 | 72 | 37.9 | 0.46 |
| Ex. 5 | 90 | 47.3 | 90 | 42.6 | 52.7 | 72 | 37.9 | 1.12 |

As seen from Table 3, the ratio of PC/swellable is higher than for the comparative examples. It is considered that a high ratio of PC/swellable of at least 0.30 is required to achieve a combination of good viscosity and a good swelling index.

**Table 4 Basal spacing and intensity of a characteristic diffraction peak of the X-ray diffractograms of the modified clays (MC)**

| **Grade** | **d001 (in Angstroms)** | **Intensity of the peak** |
|---|---|---|
| Comp. Ex.1 | 28,0 | 7000 |
| Comp. Ex.2 | 29,3 | 7500 |
| Comp. Ex.3 | | |
| Ex.4 | 28.4 | 10000 |
| Ex.5 | 27.2 | 14000 |

It is shown in Table 4 that the organoclays of Ex. 4 and Ex.5 have an important interlayer distance (d001), that means the modified clay has a paraffin type configuration in the interlayer space, as described in Christidis, The concept of layer charge of smectites and its implications for important smectite-water properties, EM U Notes in Mineralogy, 11, 2011.

In addition, the intensity of the peak indicates a homogeneous distribution of the phospholipid between the sheets of the clay. The combination of the two parameters traduces a good swelling of the organoclay.

## Claims

1. A modified clay comprising a clay and a phospholipid,
wherein the clay comprises a swellable clay; and the modified clay comprises less than about 20 cmol/kg of cations not including sodium cations.

2. The modified clay according to claim 1, wherein the modified clay has an X-ray diffractogram comprising at least the following characteristic diffraction peak:
a basal spacing (001) located at a distance of the order of 25-40 Å, representing said swelling mineral phase, which peak has an intensity of at least 8000.

3. The modified clay according to claim 1 or 2, wherein the modified clay has a Cation Exchange Capacity (CEC) of no more than 50 cmol/kg.

4. The modified clay according to any preceding claim, wherein the clay comprises a swellable clay in an amount of about 70 wt.% or more based on the total weight of clay.

5. The modified clay according to any preceding claim, wherein the swellable clay is selected from smectite clay, a trioctahedral smectite clay or a dioctahedral smectite clay, wherein the trioctahedral smectite clay is selected from one or more of saponite, hectorite or laponite and/or wherein the dioctahedral smectite clay is selected from one or more of montmorillonite, bentonite, nontronite, or beidelleite.

6. The modified clay according to any preceding claim, wherein the phospholipid is between two sheets of clay.

7. The modified clay according to any preceding claim, where the phospholipid is selected from a cationic phospholipid, a zwitterionic phospholipid, a non-ionic phospholipid or combinations thereof, and/or where the phospholipid is phosphatidylcholine, phosphatidylethanolamine, or phosphatidylserine.

8. The modified clay according to any preceding claim, wherein the phospholipid is from a natural source, optionally wherein the phospholipid is from lecithin.

9. The modified clay according to any preceding claim, wherein the modified clay comprises less than 10 wt.% of Fe₂O₃, based on the total weight of the modified clay.

10. A method of preparing a modified clay using an organophylization process, comprising the treatment of clay with a phospholipid, wherein the clay comprises swellable clay, and wherein the weight ratio of phospholipid to swellable clay is between about 0.30 to about 1.20, optionally wherein the organophylization process is carried out by mixing the clay and the phospholipid; and heat treating the mixture at a temperature of about 30 °C to about 50 °C for at least 1 h.

11. A modified clay obtained according to the method of claim 10.

12. Use of the modified clay according to any one of claims 1 to 9 or claim 11 as a thickener.

13. The use according to claim 12 in a cosmetic formulation, optionally wherein the cosmetic formulation is foundation, nail polish, deodorant, antiperspirant, sun care product, mascara, eyeliner and/or lipstick.

14. A cosmetic composition comprising the modified clay of any one of claims 1 to 9 or claim 11.

15. The cosmetic composition of claim 14 for use in a cosmetic formulation, optionally wherein the cosmetic formulation is foundation, nail polish, deodorant, antiperspirant, sun care product, mascara, eyeliner and/or lipstick.
